# EUROPEAN PATENT SPECIFICATION

(11) **EP 2 201 379 B1**
(45) Date of publication and mention of the grant of the patent: **21.01.2015**
(21) Application number: 08763745.0
(22) Date of filing: 21.05.2008
(51) Int. Cl.: G01N 33/58, G01N 33/68

(54) **IN VIVO LABELING OF MAMMALIAN CONE PHOTORECEPTORS BY INTRAVITREAL INJECTION OF FLUORESCENTLY TAGGED PEANUT AGGLUTININ**
IN-VIVO-MARKIERUNG VON ZAPFEN-PHOTOREZEPTOREN VON SÄUGERN DURCH INTRAVITREALE INJEKTION VON MIT EINEM FLUORESZENZ-TAG VERSEHENEN ERDNUSS-AGGLUTININ
MARQUAGE IN VIVO DE PHOTORÉCEPTEURS CÔNES DE MAMMIFÈRE PAR INJECTION INTRAVITRÉENNE D'AGGLUTININE D'ARACHIDE MARQUÉE PAR FLUORESCENCE

(30) Priority: 11.10.2007 IN DE21362007
(43) Date of publication of application: 30.06.2010
(73) Proprietor: Department of Biotechnology, New Delhi 110 003 (IN); National Brain Research Centre, Haryana 122 050 (IN)
(72) Inventor: DHINGRA, Narender, K., Haryana 122 050 (IN); VIDHYASANKAR, K., Haryana 122 050 (IN)
(74) Representative: Tuxworth, Pamela M.
(86) International application number: PCT/IN2008/000321
(87) International publication number: WO 2009/047778

(56) References cited:
- KRISHNAMOORTHY, V. ET AL: "In Vivo Labeling of Mammalian Cone Photoreceptors by Intravitreal Injection of Fluorescently Tagged Peanut Agglutinin", Journal of Vision, vol. 7, no. 5, ABST.75, 31 December 2007 (2007-12-31), XP002637797, DOI: 10.1167/7.15.75 Retrieved from the Internet: URL:http://www.journalofvision.org/content /7/15/75 [retrieved on 2009-12-10]
- ROGERS BRIAN S ET AL: "Differential sensitivity of cones to iron-mediated oxidative damage", INVESTIGATIVE OPHTHALMOLOGY & VISUAL SCIENCE, [Online] vol. 48, no. 1, 1 January 2007 (2007-01-01), pages 438-445, XP009127064, ASSOCIATION FOR RESEARCH IN VISION AND OPHTHALMOLOGY, US ISSN: 0146-0404, DOI: 10.1167/IOVS.06-0528 Retrieved from the Internet: URL:http://www.iovs.org/>
- VON SCHANTZ M ET AL: "Difference in PNA label intensity between short- and middle- wavelength sensitive cones in the ground squirrel retina", INVESTIGATIVE OPHTHALMOLOGY & VISUAL SCIENCE, vol. 34, no. 13, 1 December 1993 (1993-12-01), pages 3641-3645, XP003026568, ASSOCIATION FOR RESEARCH IN VISION AND OPHTHALMOLOGY, US ISSN: 0146-0404
- TAKUMI K ET AL: "In vivo lectin-binding of photoreceptors and interphotoreceptor matrix in rat", JAPANESE JOURNAL OF OPHTHALMOLOGY, vol. 35, no. 1, 1 January 1991 (1991-01-01), pages 16-22, XP009127068, MARUZEN CO., LTD., TOKYO, JP ISSN: 0021-5155
- KRISHNAMOORTHY VIDHYASANKAR ET AL: "Intravitreal injection of fluorochrome-conjugated peanut agglutinin results in specific and reversible labeling of mammalian cones in vivo", INVESTIGATIVE OPHTHALMOLOGY & VISUAL SCIENCE, [Online] vol. 49, no. 6, 1 June 2008 (2008-06-01), pages 2643-2650, XP009127062, ISSN: 0146-0404, DOI: 10.1167/IOVS.07-1471 Retrieved from the Internet: URL:http://www.iovs.org/>
- 'OSA vision meeting', 2007 article KRISHNAMOORTHY, V. ET AL.: 'In Vivo Labeling of Mammalian Cone Photoreceptors by Intravitreal Injection of Fluorescently Tagged Peanut Agglutinin'
- ROGERS BS ET AL.: 'Differential sensitivity of cones to iron-mediated oxidative damage.' INVEST OPHTHALMOL VIS SCI. vol. 48, no. 1, January 2007, pages 438 - 45, XP009127064
- SZÉL A ET AL.: 'Difference in PNA label intensity between short- and middle- wavelength sensitive cones in the ground squirrel retina.' DIFFERENCE IN PNA LABEL INTENSITY BETWEEN SHORT- AND MIDDLE- WAVELENGTH SENSITIVE CONES IN THE GROUND SQUIRREL RETINA. vol. 34, 1993, pages 3641 - 3645, XP003026568
- TAKUMI K ET AL.: 'In vivo lectin-binding of photoreceptors and interphotoreceptor matrix in rat.' JPN J OPHTHALMOL. vol. 35, no. 1, 1991, pages 16 - 22, XP009127068
- KRISHNAMOORTHY V ET AL.: 'Intravitreal injection of fluorochrome-conjugated peanut agglutinin results in specific and reversible labeling of mammalian cones in vivo.' INVEST OPHTHALMOL VIS SCI. vol. 49, no. 6, June 2008, pages 2643 - 50, XP009127062

## Description

### Field of Invention

This invention relates to a novel, method of in vivo labeling of outer and inner segments, and pedicles of mammalian cone photoreceptors by intravitreal injection of fluorescently tagged peanut agglutinin. This has clinical implications for early and confirmed diagnosis, and status assessment of retinal degenerative diseases involving photoreceptors in humans.

### Background of the invention

Retinal degenerative disease such as Retinitis Pigmentosa (RP) and Age-Related Macular Degeneration (AMD) are characterized by progressive degeneration and loss of photoreceptors. These diseases affect a large proportion of population (RP incidence: 1 in ∼4000; AMD incidence¹ : ∼4% at 43-54 years of age, ∼46%_ above 75 years), and are among the leading causes of blindness. Recent data on Indian population suggest that the prevalence of retinal degenerative diseases in India is similar to that in Western countries^{2, -3, 4} since these diseases initially cause only partial loss of vision, mostly in dim light, they may not be diagnosed early on. This problem is further confounded by the fact that there is no technique currently available that would allow a direct and confirmed diagnosis of the photoreceptor degeneration. As a result, these diseases are typically diagnosed based on personal and family medical history, and a group of nonspecific signs and symptoms, such as night blindness, visual field defects, bone-spicule pigmentation, drusen, arteriolar attenuation, macular hemorrhage and abnormal electroretinogram etc. If the disease is diagnosed early, some of the newer treatment strategies may prove to be effective. This is particularly important because loss of photoreceptors in these diseases initiates a series of other potentially irreversible changes, including remodeling of the retinal circuitry.^{5, 6}

It is possible to view larger retinal neurons, e.g., ganglion cells (10-30 µm) that are fluorescently labeled in a living animal.⁸ However, most retinal degenerative diseases primarily affect photoreceptors which are much smaller (2-10 µm in diameter) and cannot be directly viewed with any routine clinical method partly because they have not been shown to take any non-toxic fluorescent marker in vivo. Even advanced ophthalmic techniques, such as Optical Coherence Tomography (OCT) and scanning Laser Ophthalmoscopy (SLO) cannot resolve retina at the level of photoreceptors. Adaptive Optics, which employs a series of mathematical calculations to compensate fro the optic blur in the eye, does allow detection of photoreceptors in living human retina⁹, but has limitations in terms of axial and transverse resolution, and can not differentiate between specific parts of a cone.

It has been known for may years that several plant lectins bind to interphotoreceptor matrix around specific regions of photoreceptors in vitro. Peanut Agglutinin (PNA) is known to bind to cone interphotoreceptor matrix to label selectively cone outer and inner segments, and cone pedicles. When conjugated with horseradish peroxides (HRP), and injected subretinally into rats in vivo, PNA has been shown to selectively bind to cone photoreceptors.¹⁰ However, HRP cannot be excited to emit any light and thus may not be detected better than unstained photoreceptors. More importantly, to render HRP visible involves steps that cannot be carried out in vivo, e.g., use of toxic compounds diaminobenzidine. Furthermore, subretinal injection can cause complications, including retinal detachment and damage, and therefore cannot be done routinely in humans. Together, these reasons probably explain why subretinal injection of PNA has not been attempted in animals or humans for any clinical use.

We asked if PNA conjugated with a fluorescent probe when administered intravitreally in vivo could penetrate through inner limiting membrane and several layers of retinal cells to selectively label mammalian cones. Fluorescent probes such as Fluorescein are routinely applied locally in the eye or injected systemically into the blood stream in a variety of ophthalmic conditions in humans, and are thus considered safe. Here inventors report a relatively simple and safe method of selectively labeling specific parts of a cone in a living animal, which may not only augment the recent advances in optical techniques in detecting single photoreceptors in vivo, but can also be potentially employed in routine ophthalmic practice to diagnose and assess the progression of some of the retinal degenerative diseases. To our knowledge there is no report that PNA or any other plant lectin conjugated with a fluorescent probe can be injected intravitreally for visualization of retinal cells in vivo.

One previous invention (Patent No. Wo 2005054447) describes a method for producing retinal cells in culture that can potentially be used for cell transplantation therapies. Some of these retinal cells are claimed to bind to peanut agglutinin. This invention is an in vitro method of producing specialized retinal cells, whereas the present invention is an in vivo method of injecting peanut agglutinin into the eye which may be used for diagnosing retinal degenerative diseases.

Another invention (Patent No.Wo 2005103232) describes a method for isolating and purifying cone photoreceptors using agglutinin in vitro, but the present invention is an in vivo method of injecting peanut agglutinin into the eye, which may be used for diagnosing retinal degenerative diseases.

Another invention (Patent No. Wo 2003039346) describes a new interphotoreceptor compound that may be used to prevent or treat photoreceptor death by gene therapy, whereas this invention is an in vivo method of injecting peanut agglutinin into the eye where it binds specifically to the interphotoreceptor matrix around cone photoreceptors, which may be used for diagnosing retinal degenerative diseases.

### Object of the Invention

The object of this invention is to develop a method for selectively labeling specific parts cone photoreceptors in vivo, which would allow their direct visualization and assessment of their degeneration across entire retina in a living animal or human.

Other object is to develop a method which could enable clinicians to make an early and confirmed diagnosis of retinal degenerative diseases involving photoreceptors.

Another object is to develop a method of in vivo labeling of specific photoreceptors by intravitreal injection of Peanut Agglutinin.

Yet another object is to develop a method of in vivo labeling of specific parts of cone photoreceptors by intravitreal injection.

Other object is to develop a method using Peanut Agglutinin wherein the fluorescent probe conjugated to Peanut Agglutinin could be used to visualize the presence or absence of cones in vivo.

Another object is to develop a method to assess the location and extent of cone degeneration in vivo.

Yet another object is to develop a method that has potential to make early and confirmed diagnosis of retinal degenerative diseases involving photoreceptors and also can provide an assessment of the disease progression.

### Statement of Invention

According to this invention there is provided a method for specifically labeling outer and inner segments and pedicles of mammalian cone photoreceptors in vivo comprising intravitreal injection of 2 µl for adult anesthetized mouse (C57BL/6 J), 10 µl for guinea pig (Ducan Hartley) of 20 µl for monkey (Macaca mulatta) of PNA conjugated with a fluorescent probe (Rhodamine or Fluorescein) at a concentration of 0.005%, 0.01%, 0.02%, 0.05% or 0.5% (only 0.5% for guinea pig and monkey) with a glass electrode having a tip diameter of ∼-1 [mu]m or an insulin syringe posterior to the limbus into the vitreous of the eye. Removing the eye under anesthesia at various time points (mouse: 0.5, 1, 2, 4, and 8 hours; 1, 2, 4, 7 and 10 days; 1 and 3 months guinea pig and monkey: 30 minutes) after the PNA injection. Hemisecting and fixing the posterior eye-cup in 4% paragormaldehyde for 1 hour at 4°C flat mounting the retina on a glass slide or cryosectioning and observing PNA labeling with a fluorescence microscope.

The invention provides PNA conjugated with a fluorescent probe selected from Rhodamine or Fluorescein for use in a method of specifically labelling *in vivo* outer and inner segments and pedicles of mammalian cone photoreceptors.

The invention further provides the use of PNA conjugated with a fluorescent probe selected from Rhodamine or Fluorescein in the manufacture of a diagnostic for a retinal degenerative disease, wherein said diagnostic specifically labels *in vivo* outer and inner segments and pedicles of mammalian cone photoreceptors.

### Brief description of the accompanying drawings

**Figure 1:** Intravitreally injected, fluorescently tagged Peanut Agglutinin results in selective labeling of cone photoreceptors in live mouse, guinea pig and monkey. Peanut Agglutinin (PNA, 0.5%) conjugated with Rhodamine was injected intravitreally into anesthetized mouse (A, B), guinea pig (C, D) or monkey (E, F). Retinas removed after 30 minutes show specific labeling of cone photoreceptors across entire retina in a flatmount preparation. A, C E: central retina; B, D, F: peripheral retina. Scale bar = 10 µm.
**Figure 2:** Intravitreally injected, fluorescently tagged Peanut Agglutinin results in selective labeling of cone outer and inner segments and cone pedicles in vivo. Peanut Agglutinin (PNA, 0.05%) conjugated with Fluorescein was injected intravitreally into anesthetized mouse. Sections of retina removed after 30 minutes show specific labeling of cone outer (arrow) and inner (black arrowhead) segment and cone pedicles (white arrowhead), as has been reported for in vitro staining. Scale bar = 10 µm.

### Detailed description of the invention

Present invention where inventors are able to specifically label cones with intravitreal injection of PNA-Fluorescein or PNA-Rhodamine in a live animal could enable direct viewing of these photoreceptors, and thus allow not only confirmed diagnosis of retinal degenerative diseases involving photoreceptors, but also an assessment of the disease progression in humans. Since intravitreal PNA injection resulted in labeling of live cones in several mammalian species, including a non-human primates and that PNA is known to stain cones in postmortem human retina⁷, it is highly likely that it will work similarly in living humans. The probe Fluorescein, for example, can be excited at a wavelength of ∼495 nm and the emitted light of ∼520 nm can be detected with a variety of optical devices. The invention of this relatively simple and safe technique should enable clinicians to make early and confirmed diagnosis of some of the retinal degenerative diseases, which in turn would allow introducing suitable therapeutic measures at more appropriate stage of disease progression.

**Methods:** All experiments were approved by Institutional Animal Ethics Committee of National Brain Research Centre, and Committee for the Purpose of Control and Supervision of Experiments on Animals. Adult mouse (C57BL/6J), guinea pig (Duncan Harley) and monkey (Macaca mulatta) were used in the present study. The animals were anesthetized with ketamine and xylazine. A 2 µl (mouse), 10 µl (guinea pig) or 20 µl (monkey) of PNA conjugated with a fluorescent probe (Rhodamine or Fluorescein) at a concentration of 0.005%, 0.01%, 0.02%, 0.05% , or 0.5% for mouse; 0.5% for guinea pig and monkey was injected with a glass electrode having a tip diameter of ∼ 1 µm or a standard insulin syringe posterior to the limbus into the vitreous of the eye. The eye was removed under anesthesia at various time-points after the PNA injection (Mouse: 0.5, 1, 2, 4, and 8 hours: 30 minutes), hemisected, and the posterior eye-cup fixed in 4% paraformaldehyde for 1 hour at 4°C. Retina was flat-mounted cryosectioned, and PNA labeling was observed with a fluorescence microscope. TUNEL assay was carried out to see if PNA caused any apoptosis of retinal cells. Standard immunofluorescence experiments were carried out for some of the cell-specific proteins (PSD-95, PKC, syntaxin-1, SMI-32) to look for any qualitative changes in retinal neurons. Gross visual function of the PNA-injected animal was tested under photopic and scotopic light conditions in a Visual Cliff Test after 8 hours of PNA injection.

**Results:** PNA conjugated with either Rhodamine or Fluorescein, when injected intravitreally in vivo was found to selectively label cone photoreceptors across entire retina. All mammalian species tested here showed the specific labeling (Fig. 1). Like the in vivo binding, PNA specifically labeled cone outer and inner segments, and cone pedicles (Fig. 2). The lowest concentration of PNA that showed complete and consistent labeling of mouse cones was 0.05%_lower concentrations also labeled cones but focally and inconsistently. At this concentration the mouse retina showed cone labeling for at least 10 days after the intravitreal injection. However, retina removed 30 days after the injection did not show any labeling, implying that PNA completely washed out at some point between 10 and 30 days. When retinal sections after complete washout were re-stained with PNA, they again showed specific labeling of cone outer and inner segment and cone pedicles, implying that intravitreally injected PNA did not alter the binding capacity of cone interphotoreceptor matrix for PNA. TUNEL staining of the retina removed either 30 minutes or 3 months after PNA injection was negative, implying that PNA or the fluorescent probe did not have any apoptotic effect on retinal cells for up to 3 months. Retinal neurons immunostained for PSD-95, PKC, syntaxin-1 and SMI-32 in the PNA-injected retina were indistinguishable from the normal retina, suggesting that photoreceptors, rod bipolar cells, amacrine cells and ganglion cells wee normal. Visual Cliff test performed 8 hours after the PNA injection showed normal visual behavior.

### Reference List

1. Klein R, Klein BE, Tomany Sc, Meuer SM, Huang GH (2002) Ten-year incidence and progression of age-related maculaopathy: The Beaver Dam Eye Study. Ophthalmol. 10: 1767-1779.
2. Nirmalan PK, Katz J, Robin AL, Tielsch JM, Namperumalsamy P, Kim R, Narendran V, Ramakrishnan R, Krishnadas R, Thulsiraj RD, Suan E (2004) Prevalence of vitreoretinal disorders in a rural population of southern India. Arch. Ophthalmol. 122: 581-586.
3. Krishnaiah S, Das T, Nirmalan PK, Nutheti Rm Shamanna BR, Rao GN, Thomas R (2005) Risk factors for age-related macular degeneration: Findings from the Andhra Pradesh eye disease study in south India. Invest. Ophthalmol. Vis. Sci., 46: 4442-4449.
4. Gupta SK, Murthy GVS, Morrison N, Price GM, Dherani M, John N, Fletcher AE, Chakravarthy U (2007) Prevalence of early and late age- related macular degeneration in a rural population in northern India: The INDEYE feasibility study. Invest. Ophthalmol. Vis. Sci. 48: 1007-1011.
5. Jones BW, Watt CB, Marc RE (2005) Retinal remodeling. Clin. Exp. Optom. 88: 282-291.
6. Sullivan RKP, WoldeMussie E. Pow DV (2007) Dentritic and synaptic Plasticity of neurons in the human age-related macular degeneration retina. Invest. Ophthalmol. Vis. Sci. 48: 2782-2791.
7. Bopp S, El-Hifnawi S, Laqua H (1994) the photoreceptor cells and retinal pigment epithelium of normal and is eased human retinas expres different glycoconjugates. Ger. J. Ophthalmol. 3: 27-36.
8. Cordeiro MF, Guo L, Luong V, Harding G, Wang W, Jones HE, Moss SE, Sillito AM, Fitzke FW (2004) Real-time imaging of single nerve cell apoptosis in retinal neurodegeneration. Proc. Natl. Acad. Sci. 101: 13352-13356.
9. Roorda A, Williams DR (1999) The arrangement of the three cone classed in the living human eye. Nature, 397: 520-522.
10. Takumi K, Uehara F (1991) In vivo lectin-binding of photoreceptors and interphotoreceptor matrix in rat. Jpn. J. Ophthalmol. 35: 16-22.

It is to be noted that the formulation of the present invention is susceptible to modifications, adaptations and changes by those skilled in the art. Such variant formulations are intended to be within the scope of the present invention which is further set forth under the following claims:-

## Claims

1. PNA conjugated with a fluorescent probe selected from Rhodamine or Fluorescein for use in a method of specifically labelling *in vivo* outer and inner segments and pedicles of mammalian cone photoreceptors by intravitreal injection.

2. PNA conjugated with a fluorescent probe for the use as claimed in claim 1, wherein the concentration is at a range between 0.005% and 0.5%.

3. PNA conjugated with fluorescent probe for the use as claimed in claim 1, wherein the method comprises:
(i) intravitreal injection of 2 µl for adult anesthetized mouse (C57BL/6 J), 10 µl for guinea pig (Duncan Hartley) or 20 µl for monkey (Macca malatta) of PNA conjugated with a fluorescent probe (Rhodamine or Fluorescein) at a concentration of 0.005%, 0.01%, 0.02%, 0.05% or 0.5% (only 0.5% for guinea pig and monkey) with a glass electrode with tip diameter of ∼ 1 µm or an insulin syringe posterior to the limbus into the vitreous of the eye,
(ii) removing the eye under anesthesia at various time points (mouse: 0.5, 1, 2, 4 and 8 hours, 1, 2, 4, 7 and 10 days; 1 and 3 months; guinea pig and monkey; 30 minutes) after the PNA injection,
(iii) hemisecting and fixing the posterior eye-cup in 4% paraformaldehyde for 1 hour at 4°C,
(iv) flat mounting the retina on a glass slide or cryosectioning, and observing PNA labelling with a fluorescence microscope.

4. The use of PNA conjugated with a fluorescent probe selected from Rhodamine or Fluorescein in the manufacture of a diagnostic for a retinal degenerative disease, wherein said diagnostic specifically labels *in vivo* outer and inner segments and pedicles of mammalian cone photoreceptors.

5. The use of PNA conjugated with a fluorescent probe as claimed in claim 4, wherein said retinal degenerative disease is retinitis pigmentosa (RP) or age-related macular degeneration (AMD).

6. The use of PNA conjugated with a fluorescent probe as claimed in claim 4 wherein the concentration is at a range between 0.005% and 0.5%.

7. The use of PNA conjugated with fluorescent probe as claimed in claim 4 wherein the diagnostic is used in a method which comprises:
(i) intravitreal injection of 2 µl for adult anesthetized mouse (C57BL/6 J), 10 µl for guinea pig (Duncan Hartley) or 20 µl for monkey (Macca malatta) of PNA conjugated with a fluorescent probe (Rhodamine or Fluorescein) at a concentration of 0.005%, 0.01%, 0.02%, 0.05% or 0.5% (only 0.5% for guinea pig and monkey) with a glass electrode with tip diameter of ∼ 1 µm or an insulin syringe posterior to the limbus into the vitreous of the eye,
(ii) removing the eye under anesthesia at various time points (mouse: 0.5, 1, 2, 4 and 8 hours, 1, 2, 4, 7 and 10 days; 1 and 3 months; guinea pig and monkey; 30 minutes) after the PNA injection,
(iii) hemisecting and fixing the posterior eye-cup in 4% paraformaldehyde for 1 hour at 4°C,
(iv) flat mounting the retina on a glass slide or cryosectioning, and observing PNA labelling with a fluorescence microscope.

## Patentansprüche

1. PNA, konjugiert mit einer fluoreszierenden Sonde, die aus Rhodamin oder Fluorescein ausgewählt ist, zur Verwendung in einem Verfahren zum spezifischen Markieren *in vivo* von äußeren und inneren Segmenten und Pedunkuli von Säugetier-Konusphotorezeptoren durch intravitreale Injektion.

2. PNA, konjugiert mit einer fluoreszierenden Sonde zur Verwendung, wie in Anspruch 1 beansprucht, wobei die Konzentration in einem Bereich zwischen 0,005% und 0,5% liegt.

3. PNA, konjugiert mit einer fluoreszierenden Sonde, zur Verwendung, wie in Anspruch 1 beansprucht, wobei das Verfahren umfasst:
(i) intravitreale Injektion von 2 µl für erwachsene anästhesierte Maus (C57BL/6 J), 10 µl für Meerschweinchen (Duncan Hartley) oder 20 µl für Affe (Macca malatta) an PNA, konjugiert mit einer fluoreszierenden Sonde (Rhodamin oder Fluorescein) mit einer Konzentration von 0,005%, 0,01%, 0,02%, 0,05% oder 0,5% (nur 0,5% für Meerschweinchen und Affe) mit einer Glaselektrode mit einem Spitzendurchmesser von ∼ 1 µm oder einer Insulinspritze hinter dem Hornhautrand in den Glaskörper des Auges,
(ii) Entfernen des Auges unter Anästhesie zu verschiedenen Zeitpunkten (Maus: 0,5, 1, 2, 4 und 8 Stunden, 1, 2, 4, 7 und 10 Tage; 1 und 3 Monate; Meerschweinchen und Affe: 30 Minuten) nach der PNA-Injektion;
(iii) Hämisektion und Fixierung des hinteren Augenschälchens in 4% Paraformaldehyd für 1 Stunde bei 4°C,
(iv) Flachmontieren der Retina an einem Objektträger oder Kryoschneiden und Betrachten der PNA-Markierung mit einem Fluoreszenzmikroskop.

4. Verwendung von PNA, konjugiert mit einer fluoreszierenden Sonde, die aus Rhodamin oder Fluorescein ausgewählt ist, in der Herstellung eines Diagnostikums für eine degenerative Retinaerkrankung, wobei das Diagnostikum spezifisch *in vivo* äußere und innere Segmente und Pedunkuli von Säugetier-Konusphotorezeptoren markiert.

5. Verwendung von PNA, konjugiert mit einer fluoreszierenden Sonde, wie in Anspruch 4 beansprucht, wobei die degenerative Retinaerkrankung Retinitis pigmentosa (RP) oder altersbedingte Makuladegeneration (AMD) ist.

6. Verwendung von PNA, konjugiert mit einer fluoreszierenden Sonde, wie in Anspruch 4 beansprucht, wobei die Konzentration in einem Bereich zwischen 0,005% und 0,5% liegt.

7. Verwendung von PNA, konjugiert mit einer fluoreszierenden Sonde, wie in Anspruch 4 beansprucht, wobei das Diagnostikum in einem Verfahren verwendet wird, das umfasst:
(i) intravitreale Injektion von 2 µl für erwachsene anästhesierte Maus (C57BL/6 J), 10 µl für Meerschweinchen (Duncan Hartley) oder 20 µl für Affe (Macca malatta) an PNA, konjugiert mit einer fluoreszierenden Sonde (Rhodamin oder Fluorescein) mit einer Konzentration von 0,005%, 0,01%, 0,02%, 0,05% oder 0,5% (nur 0,5% für Meerschweinchen und Affe) mit einer Glaselektrode mit einem Spitzendurchmesser von ∼ 1 µm oder einer Insulinspritze hinter dem Hornhautrand in den Glaskörper des Auges,
(ii) Entfernen des Auges unter Anästhesie zu verschiedenen Zeitpunkten (Maus: 0,5, 1, 2, 4 und 8 Stunden, 1, 2, 4, 7 und 10 Tage; 1 und 3 Monate; Meerschweinchen und Affe: 30 Minuten) nach der PNA-Injektion;
(iii) Hämisektion und Fixierung des hinteren Augenschälchens in 4% Paraformaldehyd für 1 Stunde bei 4°C,
(iv) Flachmontieren der Retina an einem Objektträger oder Kryoschneiden und Betrachten der PNA-Markierung mit einem Fluoreszenzmikroskop.

## Revendications

1. Agglutinine d'arachide conjuguée avec un marqueur fluorescent, choisi parmi la rhodamine et la fluorescéine, pour utilisation dans un procédé de marquage spécifique *in vivo* des segments externes et internes et des pédicules de photorécepteurs cônes de mammifère par injection intravitréenne.

2. Agglutinine d'arachide conjuguée avec un marqueur fluorescent pour utilisation conforme à la revendication 1, pour laquelle la concentration se situe dans l'intervalle allant de 0,005 % à 0,5 %.

3. Agglutinine d'arachide conjuguée avec un marqueur fluorescent pour utilisation conforme à la revendication 1, pour laquelle le procédé comporte les opérations suivantes :
i) faire une injection intravitréenne de 2 µL, pour une souris adulte anesthésiée (C57BL/6J), de 10 µL, pour un cobaye (Duncan Hartley), ou de 20 µL, pour un singe (Macca malatta), d'agglutinine d'arachide conjuguée avec un marqueur fluorescent (rhodamine ou fluorescéine), en une concentration de 0,005 %, 0,01 %, 0,02 %, 0,05 % ou 0,5 % (mais uniquement 0,5 % pour un cobaye ou un singe), avec une électrode en verre dont la pointe a un diamètre d'environ 1 µm ou une seringue à insuline, en arrière du limbe, dans le corps vitré de l'oeil ;
ii) enlever l'oeil sous anesthésie à divers instants (0,5 heure, 1, 2, 4 et 8 heures, 1, 2, 4, 7 et 10 jours, 1 mois et 3 mois, pour une souris ; 30 minutes pour un cobaye ou un singe) après l'injection d'agglutinine d'arachide ;
iii) faire l'hémisection de la cupule oculaire postérieure et la fixer en la laissant 1 heure dans du paraformaldéhyde à 4 %, à 4 °C ;
iv) et monter la rétine à plat sur une lame de verre ou en faire une cryosection, et observer le marquage à l'agglutinine d'arachide au moyen d'un microscope à fluorescence.

4. Utilisation de l'agglutinine d'arachide conjuguée avec un marqueur fluorescent, choisi parmi la rhodamine et la fluorescéine, dans la fabrication d'un test diagnostique de maladie rétinienne dégénérative, lequel test diagnostique permet de marquer spécifiquement *in vivo* les segments externes et internes et les pédicules de photorécepteurs cônes de mammifère.

5. Utilisation de l'agglutinine d'arachide conjuguée avec un marqueur fluorescent, conforme à la revendication 4, pour laquelle la maladie rétinienne dégénérative est la rétinite pigmentaire (RP) ou la dégénérescence maculaire liée à l'âge (DMLA).

6. Utilisation de l'agglutinine d'arachide conjuguée avec un marqueur fluorescent, conforme à la revendication 4, pour laquelle la concentration se situe dans l'intervalle allant de 0,005 % à 0,5 %.

7. Utilisation de l'agglutinine d'arachide conjuguée avec un marqueur fluorescent, conforme à la revendication 4, le test diagnostique devant être utilisé dans un procédé qui comporte les étapes suivantes :
i) faire une injection intravitréenne de 2 µL, pour une souris adulte anesthésiée (C57BL/6J), de 10 µL, pour un cobaye (Duncan Hartley), ou de 20 µL, pour un singe (Macca malatta), d'agglutinine d'arachide conjuguée avec un marqueur fluorescent (rhodamine ou fluorescéine), en une concentration de 0,005 %, 0,01 %, 0,02 %, 0,05 % ou 0,5 % (mais uniquement 0,5 % pour un cobaye ou un singe), avec une électrode en verre dont la pointe a un diamètre d'environ 1 µm ou une seringue à insuline, en arrière du limbe, dans le corps vitré de l'oeil ;
ii) enlever l'oeil sous anesthésie à divers instants (0,5 heure, 1, 2, 4 et 8 heures, 1, 2, 4, 7 et 10 jours, 1 mois et 3 mois, pour une souris ; 30 minutes pour un cobaye ou un singe) après l'injection d'agglutinine d'arachide ;
iii) faire l'hémisection de la cupule oculaire postérieure et la fixer en la laissant 1 heure dans du paraformaldéhyde à 4 %, à 4 °C ;
iv) et monter la rétine à plat sur une lame de verre ou en faire une cryosection, et observer le marquage à l'agglutinine d'arachide au moyen d'un microscope à fluorescence.
